# EUROPEAN PATENT APPLICATION

(11) **EP 2 116 203 A1**
(43) Date of publication of application: **11.11.2009**
(21) Application number: 07860014.5
(22) Date of filing: 25.12.2007
(51) Int. Cl.: A61B 17/58

(54) **TAPE FOR BONE FIXATION**

(30) Priority: 10.01.2007 JP 2007002835
(71) Applicant: Alfresa Pharma Corporation, Chuo-ku Osaka-shi Osaka 540-8575 (JP)
(72) Inventor: MORIHARA, Tsutomu, Noda-shi Chiba 270-0216 (JP); SUDO, Toshio, Noda-shi Chiba 270-0216 (JP); YAMAGUCHI, Noritoshi, Osaka-shi Osaka 540-8575 (JP)
(74) Representative: Behnisch, Werner
(86) International application number: PCT/JP2007/074785
(87) International publication number: WO 2008/084669

(57) **Abstract**

A tape made of polyester fibers is disclosed which, though a flexible tape, is not easily elongated when it is pulled. The tape is a tape which is produced by braiding polyester fibers into a tape and then subjecting it to drawing, and whose maximum Young's modulus measured on a stress-elongation curve produced as it is pulled until its elongation reaches 5% of its initial length is not less than 2 GPa.

## Description

### TECHNICAL FIELD

The present invention relates to a tape for bone fixation for use in surgical operation, and more specifically to such a tape particularly suitable for fixation of the sternum.

### BACKGROUND ART

The sternum is a longitudinal flat bone occurring at the median part of the anterior wall of the thorax, which, together with the ribs and thoracic spine, forms the thorax. In surgical treatment of lesions or injuries of the organs or tissues in the thorax, midline incision of the sternum is a standard manner of operation performed in order to secure a wide view of operation area. The sternum, once cut open, is closed again when the surgery is over to reconstruct the thorax. The sternum is closed by bringing the two laterally separated sections of the sternum to meet so that their cut surfaces overlap with each other into one, and tying them mostly with wires of metal such as stainless steel. And thus, until the two sections of the sternum has fused and unified (thus the bones are "fixed"), measures are taken to prevent the sections of the sternum from opening apart from each other. However, as a metal wire is a hard material, it is not rare that the sternum tied with metal wires suffers injuries by them, such as collapsing or cut-out, especially in the case of fragile sternum as seen in patients with osteoporosis or infant patients. Such an injury is not only a problem by itself but also will cause much greater troubles: if it occurred during surgery, it would be an obstacle to the proceeding of the surgery: and if it occurred within the body after the surgery and before the bone sections are completely fused, a space would appear between the both sections of the sternum since they now are poorly tied at the site of the injury, thus hindering fusion of the sternum. Unlike bones of the other part of the body, which one can more easily keep at rest after they are tied, the sternum is exposed to strong forces through breathing or coughing, it is important to avoid causing any injury to the sternum, while keeping it tied. On the other hand, metal wires are a material which is not so flexible. It is known that when they are twisted as things are tied with them, even a moderate twist will reduce their strength to 1/3 - 1/5, and that an excessive twist of them will cause further reduction. Wires, therefore, may be fractured during a tying operation, and after the surgery, too, in the case of a hard sternum when an excessive force is applied to them. If the wires are fractured, it will create a highly dangerous situation because the sternum, now not tied, may open apart, and further, the tips of the wires may touch the organs and tissues to injure them. For this reason, a flexible and therefore safer tape-type suture, "MERSILENE^{™} tape", which is cloth made of polyester fibers, is marketed and used, though partly, in place of metal wires (see Non-patent documents 1 and 2).

As a tape made of polyester fibers is flexible and does not have any hard surface as a metal wire, the material of the tape by itself is mild to the bones. And, at the same time, they have an advantage in that they are far less likely to cause collapsing or cut-out in the sternum at the site at which it is tied, either during the operation of tying the sternum and/or after the surgery, for the tape, as compared with a metal wire, contacts the sternum on a broader and flat surface when used to tie the sternum, and its pressure therefore is dispersed. It is also an advantage of the tape that it is flexible and less likely to be fractured by external forces. Therefore, it would be quite desirable for patients if a tape made of polyester fibers could be widely used for fixation of the sternum, in place of metal cables.

However, the above-mentioned tape on the market is used only to assist the operation of tying with metal wires. This is because that tape is easily elongated when it receives a tensile force, thus being likely to loosen when a force is applied to it after tying with it is completed, and therefore is incapable of keeping the sternum tied in a proper way. In addition, as the above-mentioned tape on the marked does not permit a tying technique called double-loop sliding-knot method, which is a useful way in tying bones, it would not be suitable for use in tying bones even if it were a material which does not loosen easily.

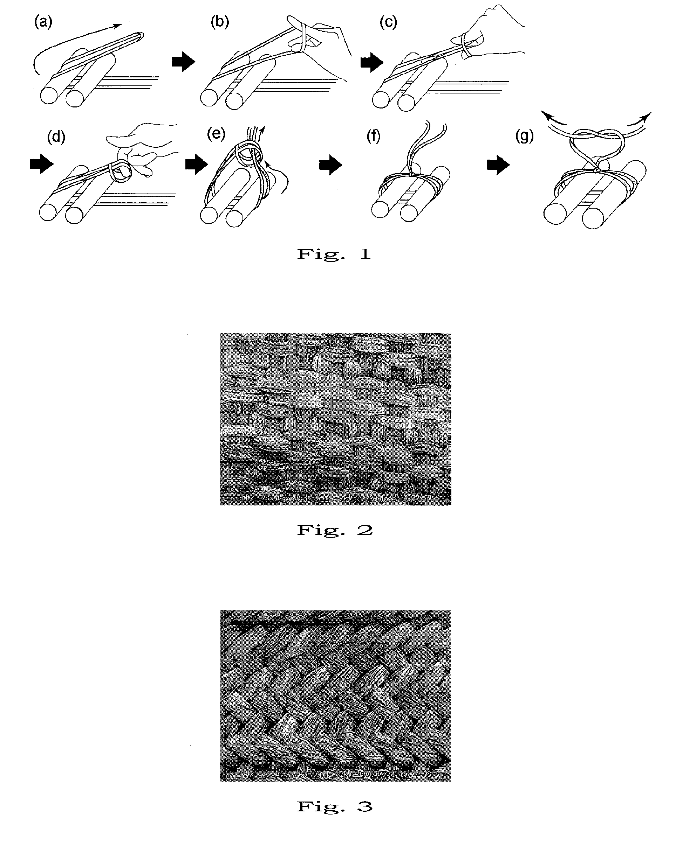

The "double-loop sliding-knot method" is a method in which, as depicted in Fig. 1, a tape, folded into two, is hung around the objects to be tied (a), a small loop then is formed by inverting the tape at the position where it is folded (b-d), and a larger loop (which encircles the object to be tied) is formed by inserting the two arms of the tape from the same direction into the small loop and drawing them (e-f). In general, tying of the sternum or other bones is performed by the following method: the two arms of the tape extending from the larger loop formed according to the double-loop sliding-knot method are tied once into a square knot (g), and then the two arms of the tape are strongly pulled from both sides so that, making use of the sliding which occurs between portions of the tape within the knot, the loop encircling the objects to be tied is gradually tightened up (hereinafter referred to as "the present tying method"). In the present tying method, it is necessary that the tape slides within the tightened knot (esp., within the square knot) so that tension is transmitted to the loop portion. So far, the only tapes whose loop can be tightened up by the present tying method have been those which are made of ultra-high molecular weight polyethylene fibers (NESPLON^{™} Cable System, and NESPLON^{™} Tape, both mftd. by Alfresa Pharma).

The above tape made of ultra-high molecular weight polyethylene fibers can be readily tightened up with a strong force by using a tightening device adapted to the tying procedure according to the present tying method (Tighting Gun [HAM], see e.g., Non-patent document 3). The above-mentioned tape made of polyester fibers, however, cannot be used in performing the present tying method, for it, once the knot (esp. square knot) is tightened up, become locked, and thus further tension is hardly transmitted to the loop any more.

A suture made of polyester fibers, "Wayolax^{™} tape with needle", which is for use in suturing and ligation of human tissues as well as in securing medial devices to the tissues, is on the market (see Non-patent document 4). The tape, which is made of polyester fibers braided into a tape-form (i.e., the fibers are interwoven in a diagonal direction), also is easily elongated by a tensile force.

Non-patent document 1: Nippon Rinsho Geka Gakkai Zasshi, Vol. 56 (No. 12): p. 1477-1480 (2001)
Non-patent document 2: ETHICON PRODUCTS CATALOG, 2004, Johnson & Johnson K.K.
Non-patent document 3: "Tighting Gun [HAM]" Pamphlet, Revised Version in August, 2006, Alfresa Pharma Corporation.
Non-patent document 4: "Wayolax tape with needle" package insert, December 12, 2005, Matsuda Ika Kogyo, Co., Ltd.

### DISCLOSURE OF INVENTION

### PROBLEM TO BE SOLVED BY THE INVENTION

Against the above-mentioned background, an objective of the present invention is to provide a tape made of polyester fibers which, though in the form of a flexible tape, is far less likely be elongated by a tensile force.
Another objective of the present invention is to provide a tape made of polyester fibers which is such a tape as above and yet allows one to firmly tie objects with it by tightening it up using the present tying method.

### MEANS TO SOLVE THE PROBLEM

The present inventors investigated starting with the current polyester tapes mentioned above. As a result, it was found that using a cloth tape of polyester fibers, which consists of longitudinal warp threads and weft threads orthogonal to the former, the above problem of easy elongation cannot be solved even if it has been drawn in advance, whereas a tape of braided polyester fibers, which by itself is easily elongated, if drawn following the braiding process of its production, unexpectedly will give a tape that is hardly elongated. The present inventors also found that though a tape produced in that manner cannot be used in the present tying method because it locks in the knot when it is being tied, this problem is solved if silicone coating is applied to it. The present invention was completed based on these findings and by further studies. Thus the present invention provides what follows.

1. A tape for bone fixation which is produced by braiding polyester fibers into a tape and then subjecting the tape to drawing.
2. The tape for bone fixation according to 1 above, wherein the maximum Young's modulus thereof which is measured on a stress-elongation curve produced as the tape is pulled until the elongation thereof reaches 5% of the initial length thereof, is not less than 2 GPa.
3. The tape for bone fixation according to 1 above which is 2-10 mm in width.
4. The tape for bone fixation according to 1 above which is coated with silicone.
5. The tape for bone fixation according to 4 above, wherein the tape, when a loop thereof is formed according to the double-loop sliding-knot method and the two arms of the tape extending from the knot of the loop are tied once into a square knot to add a further knot, and then the two arms of the tape extending from the latter knot are pulled in opposite directions with the loop encircling objects to be tied, brings about a tying force the magnitude of which is not less than 25% of the sum of the tensile force applied to either of the two arms of the tape.

### EFFECT OF THE INVENTION

Using the tape according to the present invention as defined as above, the pressure which is applied to the bone by it is dispersed, for it is made of a flexible material without having a hard surface as in the case of a metal wire, and for it is in the form of a tape and therefore, when a bone is tied with it, it contacts the bone on its tape surface, which is broader than a wire. Thus, the tape according to the present invention is much safer than a metal wire when used to tie the sternum, because it is far less likely to cause collapsing or cut-out in the sternum at the site at which it is tied, either during the process of tying the sternum or after the surgery, and because it is flexible and hardly is fractured. The tape according to the present invention is far superior in safety to a metal wire for use in the fixation not only of the sternum but also of other bones.

### BRIEF DESCRIPTION OF DRAWINGS

[FIG. 1] A schematic diagram illustrating the present tying method.
[FIG. 2] A photomicrograph of the contexture of the tape of Comparative Example 1.
[FIG. 3] A photomicrograph of the contexture of the tape of Comparative Example 2.
[FIG. 4] A photomicrograph of the contexture of the tape of Comparative Example 3.
[FIG. 5] A photomicrograph of the contexture of the tape of Comparative Example 5.
[FIG. 6] A plan view of a Tighting Gun.
[FIG. 7] A perspective view of the Tighting Gun which is being used to tie objects with a tape.
[FIG. 8] A photomicrograph of the contexture of Tape 1.
[FIG. 9] A photomicrograph of the contexture of Tape 2.

### BEST MODE FOR CARRYING OUT THE INVENTION

In the present invention, "polyester" means polyethyleneterephthalate (PET). Drawing of a tape which is produced by braiding polyester fibers (braided tape) may be carried out either at room temperature or under heating. In carrying out drawing under heating, any method for heating may be chosen as desired. A convenient method, for example, is to make part of the tape being drawn pass through a heated atmosphere. For example, heating can be done by passing the tape through air at about 100° C or 200° C for a short time (e.g., for 5 sec). When heated, a tape consisting of braided polyester fibers somewhat shrinks depending on the temperature at which it is heated. Therefore, the real amount in which the tape has been drawn is a value which consists of not only the ratio of the length of the tape that has undergone drawing to the length of the tape before drawing (i.e., apparent draw ratio), but also the shrinkage by heat that has occurred in the longitudinal direction.

In the present invention, to "braid" means to interweave multiple strands of fibers diagonally relative to the longitudinal direction of the tape, and a cord (including a tape-form one) obtained in such a manner is called a "braided cord". In this aspect, a tape-form braided cord differs from a "cloth" tape, which consists of warp threads in the longitudinal direction of the tape, and weft threads, which are orthogonal to the former.

In the present invention, a "tape" means a cord-like article whose thickness is much smaller than its width. Though there is no particular limitation as to the ratio between its width and thickness, the thickness is generally not more than 1/5 of the width. Though there is no particular limitation as to the width of a tape, either, a tape of 2-10 mm in width is generally easy to handle when used. The width, more preferably, is 3-7 mm.

In the present invention, though the term "tape for bone fixation" is used particularly preferably for fixation of the sternum, it may also be used for fixation of other bones according to a given purpose, such as the bones of a limb, the spine, etc.

In performing the measurement of Young's modulus of the tape according to the present invention, the cross sectional area of the tape is defined as that of the tape which is under no tensile force, and the cross sectional area is defined as the width times the thickness of the tape, regardless of the presence of the unfilled space between the fibers. Also in the present invention, Young's modulus of the tape, which is an index representing how the tape is less likely deformed under a tensile stress (i.e., stress/strain), is the maximum of its values measured until the tapes elongation reaches 5% of its initial length on a stress-elongation curve for the tape. This is equivalent to the maximum one of the slopes of the tangent lines drawn at each point on the corresponding portion of the stress-elongation curve for the tape. The Young's modulus of the tape according to the present invention is preferably not less than 2 GPa, and more preferably not less than 3 GPa.

In the present specification, that the tape, when a loop thereof is formed according to the double-loop sliding-knot method and a square knot is added, and, with the loop encircling objects to be tied, the two arms of the tape extending from the knot are pulled in opposite directions "brings about a tying force the magnitude of which is not less than 25% of the tensile force applied to the two arms of the tape" means that the magnitude of the force (tying force) pulling the objects to be tied toward each other is not less than 25% of the magnitude of the tensile force applied to each of the two arms of the tape (i.e., the sum of the tensile force applied each of the two arms). Typically, this can be confirmed when, under the condition that each of the two arms of the tape is pulled by a force of 230 N (460 N in total), a tying force of not less than 25% (119N) of it is brought about. In this situation, the magnitude of the tying force can be measured as the magnitude of the force applied by the loop encircling the two separate parts of the object in the direction in which they come closer to each other.

In the case where the tape for bone fixation according to the present invention is coated with silicone, there is no particular limitation as to how this is done. In a convenient method, the tape may be passed through a solution containing silicone resin at a desired concentration, e.g., about 2-10%, in a solvent (e.g., toluene), and then just allowed to dry.

### EXAMPLES

Though the present invention will be described in further detail below with reference to Comparative Examples and Examples, it is not intended that the present invention be limited to the Examples.

### [COMPARATIVE EXAMPLES]

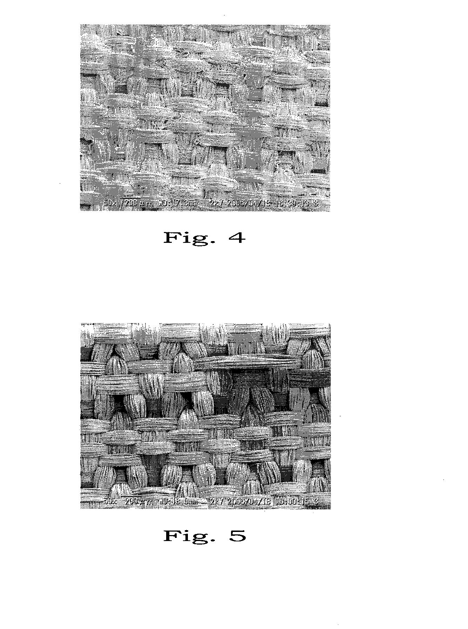

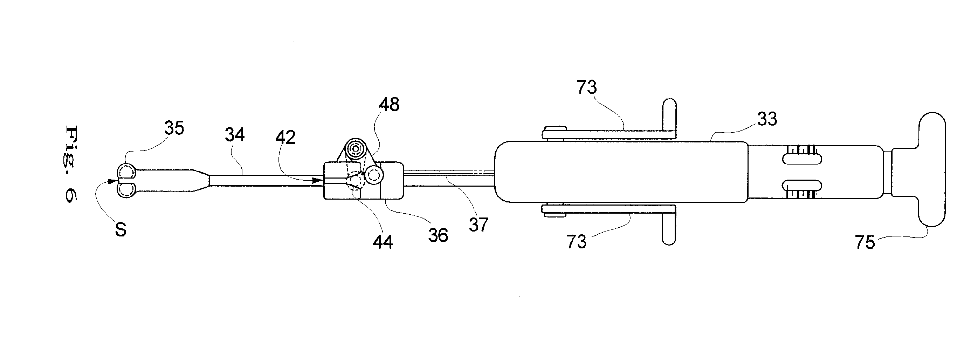

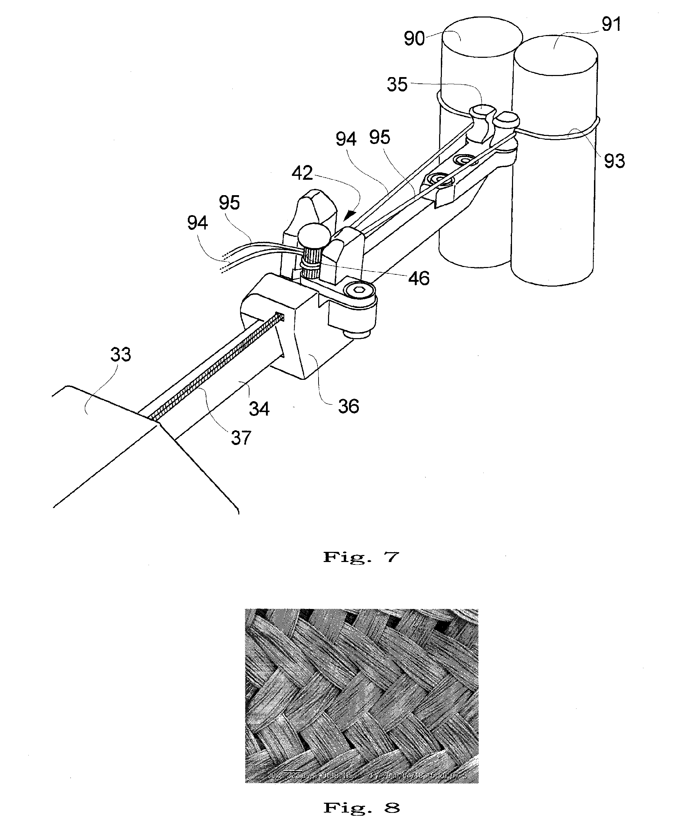

The following tapes were purchased or prepared, and then tested.
(1) Comparative Example 1: MERSILENE^{™} tape (cloth of polyester fibers, Johnson & Johnson K.K.). Fig. 2 shows a microphotograph of its contexture.
(2) Comparative Example 2: Wayolax^{™} tape with needle (braided cord of polyester fibers, Matsuda Ika Kogyo, Co., Ltd.). Fig. 3 shows a microphotograph of its contexture.
(3) Comparative Example 3: A cloth article of polyester (PET) fibers prepared in accordance with Comparative Example 1. Fig. 4 shows a microphotograph of its contexture.
(4) Comparative Example 4: A tape provided by drawing the tape of Comparative Example 3. Fig. 5 shows a microphotograph of its contexture.
Each of the tapes of Comparative Examples was measured for its width, thickness, strength (maximum tensile force which the tape can bear), and Young's modulus. With each of the tapes of Comparative Examples 1 and 2, a loop formed according to the double-loop sliding-knot method was set a tensile testing machine (Tensilon Universal testing machine RTC-1250A, mftd. by A&D Co., Ltd.), and after tying once the two arms of the tape extending from the loop into a square knot, the two arms extending the knot were pulled in opposite direction away from each other relative to the knot. A Tighting Gun [HAM] described in Non-patent document 3 was used to pull the two arms of the tape. Fig. 6 is a plan view of the device, and Fig. 7 a perspective view of the device which is being used to tie objects with a tape 93. The device, which is designed to pull together the two arms 94, 95 of the tape extending from a knot, is provided with a skiddy guide 35 so that the two arms of the tape are pulled in opposite directions away from each other around the knot. To each of the arms 94, 95 of the tape extending from the knot is applied, via a traction cable 37, a tensile force the magnitude of which is 1/2 of that of the tractional force that the device generates (the magnitude of the tractional force is determined according to a position to which the spring in the device is compressed), and the tractional force is figured out according to a scale mark read in the tension adjuster scale in the device and a predetermined correlation that exists between specific positions in the scale and the tractional force generated when the device is adjusted to one of those positions in the scale. When this is done, the direction of each of the two arms of the tape which are being pulled together after pulled away from each other in the opposite lateral directions along the guide 35, is not perfectly identical with the direction of the tractional force that the device generates. However, the difference between them is less than 5° for each of the arms. Thus, the influence of this on the magnitude of tensile force applied to the two arms of the tape was treated as negligible, because it is an increase in tensile force by only less than 0.4% (sec 5° = 1.0038). Further, as the surface of the guide 35 was smooth, any frictional force between it and the tape was negligible, too. The tying force of the tape was determined as the magnitude of the tensile force as measured on the tensile testing machine.

Table 1 presents the dimensions/physical properties, and form of each tape of Comparative Examples 1-4, and further, as for the tapes of Comparative Examples 1 and 2, the magnitude of the tying force and the proportion (%) of it to the magnitude of the tractional force as the two arms of those tapes extending from the knot are pulled with a tractional force of 314 N or 461 N using a Tighting Gun [HAM] (each of the arms receives a tensile force the magnitude of which is 1/2 of one or the other of the tractional forces, therefore, 157 N or 231 N, respectively).

### [TABLE 1]

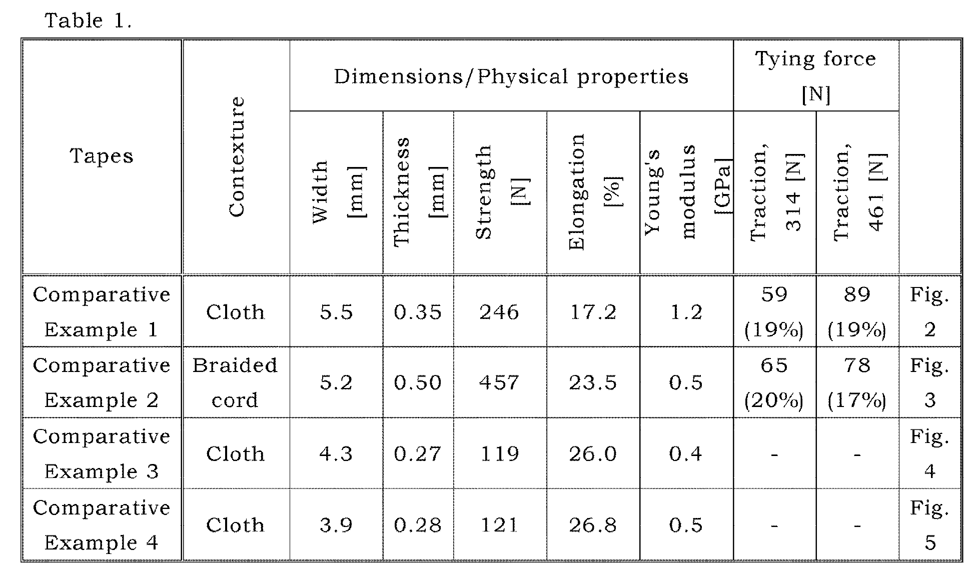

As seen in the table, the tape of Comparative Example 1 has the Young's modulus of 1.2, and it therefore is easily elongated when pulled. The tape of Comparative Example 2, a braided cord of a polyester tape, had still lower Young's modulus. The tape of Comparative Example 3 also had low Young's modulus and was easily elongated as was the tape of Comparative Example 2. The tape of Comparative Example 4, a tape provided by drawing the tape of Comparative Example 3, showed no substantial improvement with respect to its Young's modulus.

### [Example 1]

### <Tapes 1-3>

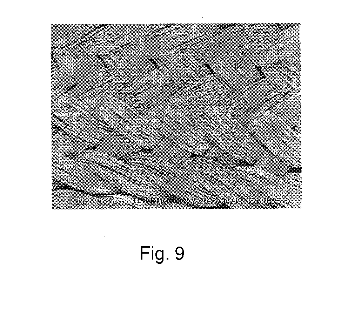

Following tapes were prepared and tested.
(1) Tape 1: A tape provided by braiding polyester (PET) fibers into a braided tape. Fig. 8 is a microphotograph showing its texture.
(2) Tape 2: A tape provide by drawing Tape 1 at room temperature. Fig. 9 is a microphotograph showing its contexture.
(3) Tape 3: A tape provided by coating Tape 2 with silicone. The coating with silicone was done by passing the tape through a solution whose silicone concentration was 10% (solvent: toluene) and then let the tape air-dry.
(4) Reference Example: NESPLON^{™} Cable System (a tape prepared by braiding ultra-high molecular weight polyethylene fibers: mftd. by Alfresa Pharma Corp.)
The same measurements as above-mentioned were done on these tapes. As for Tapes 2 and 3, the ratio was calculated of the length of a predetermined part of the tape after drawing to the length of the same part immediately before drawing (apparent draw ratio).
The results are shown in Table 2.

### [TABLE 2]

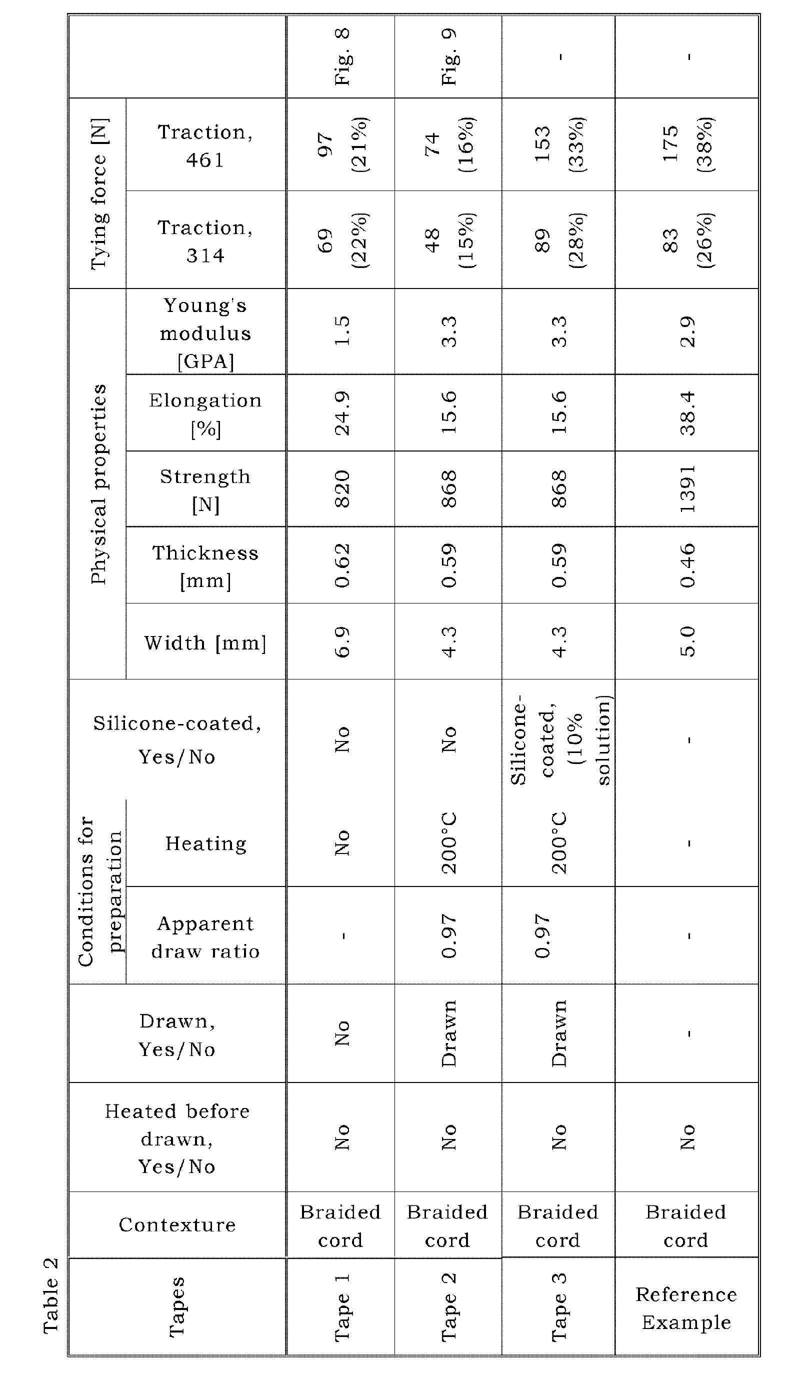

As shown in Table 2, though Tape 1, which was a braided cord prepared by just braiding polyester fibers, showed a low Young's modulus of 1.5, Tape 2, which was a tape prepared by drawing Tape 1 at room temperature, was found to exhibit a marked increase in its Young's modulus to 3.3, thus having obtained a level of Young's modulus comparable to or greater than that of the tape of Reference Example, which consisted of ultra-high molecular weight polyethylene fibers. Further, Tape 3, which was a tape prepared by coating Tape 2 with silicone, was found to transmit the tensile force very well to the loop portion as objects were being tied with it according to the double-loop sliding-knot method, thereby realizing a strong tying force. Besides, the apparent draw ratio with Tapes 2 or 3 was 0.97, a value less than 1. This is because these tapes, which had not been heated before drawing, was first heated when they were drawn, and therefore somewhat greater shrinkage occurred in them (the amount of shrinkage is not known).

### <Tapes 4-9>

As shown above, it was revealed that by subjecting a braided tape prepared by braiding polyester fibers to drawing, a tape can be obtained which has a markedly high Young's modulus and thus is not easily elongated, and that silicone-coating of thus obtained tape gives a tape which is suitable to use for tying in accordance with double-loop sliding-knot method. Thus, further studies were conducted by preparing the following tapes and subjecting them to a treatment under different conditions.
(1) Tape 4: A tape which was prepared by braiding polyester (PET) fibers into a braided tape and then subjected to drawing up to an apparent draw ratio of 1.2 in an atmosphere heated at 200° C. (The length of time during which each part of the tape passes through the heated atmosphere was about 5.5 seconds.)
(2) Tape 5: A tape which was prepared by braiding polyester (PET) fibers into a braided tape and then subjected to drawing up to an apparent draw ratio of 1.3 in an atmosphere heated at 100° C. (The length of time during which each part of the tape passes through the heated atmosphere was about 5.0 seconds.)
(3) Tape 6: A tape which was prepared by braiding polyester (PET) fibers into a braided tape and then subjected to drawing up to an apparent draw ratio of 1.3 in an atmosphere heated at 200° C. (The length of time during which each part of the tape passes through the heated atmosphere was about 5.0 seconds.)
(4) Tape 7: Tape 6 which was silicone-coated (10% solution).
(5) Tape 8: Tape 6 which was silicone-coated (6% solution).
(6) Tape 9: Tape 9 which was silicone-coated (4% solution).
   These tapes were tested as described above. The results are shown in Table 3.

### [TABLE 3]

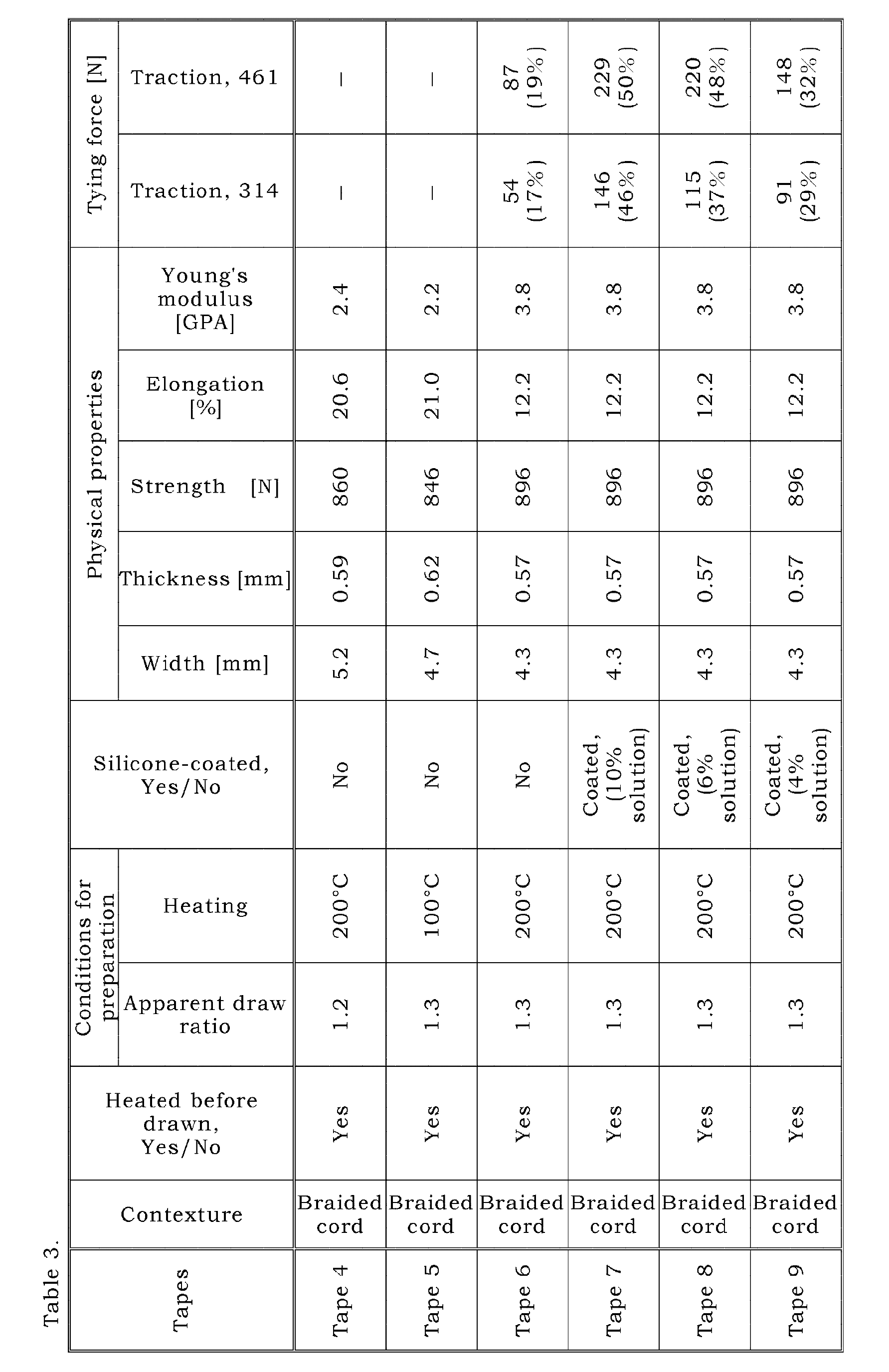

As shown in Table 3, Tape 6, a tape of braided polyester fibers, which was drawn up to an apparent draw ratio of 1.3 in an atmosphere heated at 200° C has a remarkably higher Young's modulus compared with Tape 5, a tape which was drawn up to the same draw ratio in an atmosphere heated at 100° C. However, Tape 2 shown in Table 2, which was not heated when drawn (drawn at room temperature), also exhibits a high Young's modulus which falls between them. Therefore, as indicated by these results, heating during drawing is not essential, but it would be advantageous that drawing be carried out in an atmosphere at about 200° C.

Tape 6, which was not silicone-coated, did not sufficiently transmit the tying force to the loop according to the double-loop sliding-knot method, whereas Tapes 7-9, which were prepared by coating Tape 6 with silicone, all gave sufficiently strong tying force following the tying procedure according to the double-loop sliding-knot method. This indicates that the tape prepared by drawing a braided tape consisting of braided polyester fibers, and coating it with silicone, makes it possible to employ the double-loop sliding-knot method in tying bones, a method which so far has never been applicable with tapes of polyester fibers.

### INDUSTRIAL APPLICABILITY

The present invention provides a tape for bone fixation which is far less likely than a metal wire to cause collapsing or cut-out in the sternum at the site where it is tied, either during the process of tying the sternum or after the surgery, and hardly fractured owing to its flexibility, and thus highly safe for use in fixation of the sternum.

## Claims

1. A tape for bone fixation which is produced by braiding polyester fibers into a tape and then subjecting the tape to drawing.

2. The tape for bone fixation according to claim 1, wherein the maximum Young's modulus thereof which is measured on a stress-elongation curve produced as the tape is pulled until the elongation thereof reaches 5% of the initial length thereof, is not less than 2 GPa.

3. The tape for bone fixation according to claim 1 which is 2-10 mm in width.

4. The tape for bone fixation according to claim 1 which is coated with silicone.

5. The tape for bone fixation according to claim 4, wherein the tape, when a loop thereof is formed according to the double-loop sliding-knot method and the two arms of the tape extending from the knot of the loop are tied once into a square knot to add a further knot, and then the two arms of the tape extending from the latter knot are pulled in opposite directions with the loop encircling objects to be tied, brings about a tying force the magnitude of which is not less than 25% of the sum of the tensile force applied to either of the two arms of the tape.
